Europäisches Patentamt

European Patent Office

Office européen des brevets

(11) Veröffentlichungsnummer : **0 149 460
B1**

# (12) EUROPÄISCHE PATENTSCHRIFT

(45) Veröffentlichungstag der Patentschrift :
30.12.86

(51) Int. Cl.⁴ : **C 07 C139/00, C 07 C143/58**

(21) Anmeldenummer : **85100112.3**

(22) Anmeldetag : **07.01.85**

(54) Verfahren zur Herstellung von p-Toluidin-2-sulfonsäure.

(30) Priorität : **18.01.84 DE 3401572**

(43) Veröffentlichungstag der Anmeldung :
**24.07.85 Patentblatt 85/30**

(45) Bekanntmachung des Hinweises auf die Patenterteilung : **30.12.86 Patentblatt 86/52**

(84) Benannte Vertragsstaaten :
**CH DE FR GB IT LI**

(56) Entgegenhaltungen :
**BERICHTE DER DEUTSCHEN CHEMISCHEN GESELLSCHAFT, Band 21, 1888, Seiten 1213-1219, Berlin, DE;
J.V. JANOVSKY et al.: "Über Substitutionsproducte des Paraazotoluols"
LIEBIGS ANNALEN DER CHEMIE, Band 265, 1891, Seiten 67-87, Weinheim, DE; A. CLAUS et al.: "Über die Orientirungsfolge bei der Substitution mehrfach substituirter Benzolderivate"**

(73) Patentinhaber : **BAYER AG
Konzernverwaltung RP Patentabteilung
D-5090 Leverkusen 1 Bayerwerk (DE)**

(72) Erfinder : **Blank, Heinz Ulrich, Dr.
Am Geusfelde 35
D-5068 Odenthal (DE)**
Erfinder : **Emde, Herbert, Dr.
Hardthofstrasse 13
D-5000 Köln 80 (DE)**
Erfinder : **Schimpf, Rolf, Dr.
Winand-Rossi-Strasse 40
D-5090 Leverkusen 1 (DE)**

EP 0 149 460 B1

**Beschreibung**

Die vorliegende Erfindung betrifft ein Verfahren zur Herstellung von p-Toluidin-2-sulfonsäure (5-Amino-2-methyl-benzolsulfonsäure), im folgenden kurz p-Toluidin-2-säure), genannt, durch Umsetzung von p-Toluidin in Schwefelsäure mit Oleum.

p-Toluidin-2-säure kann durch Sulfonierung von p-Nitrotoluol mit rauchender Schwefelsäure (Ann. *155*, 1 (1870) ; Rec. Trav. Chim. Pays-Bas *29*, (1910)) oder mit $SO_3$ (GB 1 164 752) und anschließender Reduktion der Nitrogruppe, beispielsweise mit Eisenpulver (BIOS-Berichte *1153*, Seite 174), hergestellt werden.

Aminoarylsulfonsäuren können ferner nach dem sogenannten « Backverfahren » aus den zugehörigen sauren Aniliniumsulfaten hergestellt werden (Helv. Chim. Acta *15*, 1372 (1932)) ; hierbei tritt jedoch die Sulfonsäuregruppe in die p-Stellung zur Aminogruppe oder bei besetzter p-Stellung in o-Stellung zur Aminogruppe ein. Aus p-Toluidin erhält man so die 2-Amino-5-methyl-benzolsulfonsäure (im folgenden als p-Toluidin-3-säure bezeichnet).

Es ist auch bereits beschrieben, das p-Toluidin mit dem doppelten Gewicht an stark rauchender $H_2SO_4$ mit 50 % $SO_3$ umzusetzen, wobei jedoch neben der gewünschten p-Toluidin-2-säure stets nennenswerte Mengen der unerwünschten p-Toluidin-3-säure gebildet werden. Erst durch Auswaschen der 3-Säure mit Wasser und anschließendem Umkristallisieren aus heißem Wasser erhält man eine 2-Säure nicht genannter Reinheit in nicht gennanter Ausbeute (Ann. *265*, 67 (1891)).

Spätere Nacharbeitungen bestätigten den Gehalt an unerwünschter 3-Säure. So wird in J. Chem. Soc. of Japan, *1975*, S. 1070-1075 die Sulfonierung durch Eintragen von p-Toluidin in die 7-fache Gewichtsmenge an 20 %igem Oleum ohne zusätzliches Lösungs- oder Verdünnungsmittel und Aufarbeitung über das Bariumsalz in das Kaliumsalz beschrieben. Die NMR-spektroskopische Analyse ergab als günstigstes Mischungsverhältnis 74 % der gewünschten 2-Saüre und 26 % der unerwünschten 3-Säure, wenn die Reaktionstemperatur bei 20° gehalten wurde. Bei steigender Reaktionstemperatur fällt der Anteil der 2-Säure zugunsten der 3-Saüre. Die isolierte Ausbeute an K-Salz beim günstigsten Verhältnis von 2- zu 3-Saüre beträgt nach Tab. 4 auf S. 1073 von J. Chem. Soc. of Japan 1975 nur 13 %.

In J. of the Chinese Chem. Soc., Vol. 4, 355 (1936) ist eine Nacharbeit der Vorschrift aus Ann. *265*, 67 (1891), jedoch mit einer größeren Menge an Oleum, beschrieben, wobei die Reaktionstemperatur 10 °C nicht erreichen durfte. Auch die hierbei erhaltene 2-Säure ist mit einer nicht beschriebenen Menge 3-Säure verunreinigt, die erst nach wiederholtem Auswaschen mit Alkohol und Umkristallisation aus Wasser entfernt werden kann, wobei Ausbeuteverluste angenommen werden müssen.

Es wurde nun überraschend festgestellt, daß man beim Übergang zu kleineren $SO_3$/p-Toluidin- und kleineren $H_2SO_4$/p-Toluidin-Verhältnissen auch bei mittleren Temperaturen den Anteil entstehender 3-Säure so weit drücken kann, daß bei Einhaltung der unter beschriebenen Aufarbeitung eine von 3-Säure praktisch freie 2-Saüre in hoher Ausbeute erhalten wird.

Die Erfindung betrifft daher ein Verfahren zur Herstellung von p-Toluidin-2-sulfonsäure durch Reaktion von $SO_3$-haltiger Schwefelsäure (Oleum) mit in Schwefelsäure gelöstem p-Toluidin, das dadurch gekennzeichnet ist, daß man zur Auflösung pro Mol p-Toluidin 1,5-10 Mol Schwefelsäure einsetzt und so viel Oleum bei 10-55 °C zugibt, daß pro Mol p-Toluidin 1-3 Mol freies $SO_3$ eingesetzt werden.

Erfindungsgemäß wird das p-Toluidin in schwefelsaurer Lösung eingesetzt. Hierbei werden 1,5-10 Mol $H_2SO_4$, bevorzugt 1,7-8 Mol $H_2SO_4$ pro Mol p-Toluidin eingesetzt. Im allgemeinen wird hierzu eine 100 %ige $H_2SO_4$ (sogenanntes Monohydrat) eingesetzt. Es lassen sich jedoch auch Schwefelsäuren mit einem geringen Wassergehalt, beispeislweise bis zu 8, bevorzugt bis zu 5 % $H_2O$, bezogen auf die Gesamtmenge der Säure, einsetzen. Diese Wassermenge verbraucht selbstverständlich einen Teil des in Form von Oleum später zuzusetzenden $SO_3$, so daß eventuell die Menge dieses $SO_3$ entsprechend dem Wassergehalt erhöht werden muß. Die Zugabe des p-Toluidins kann in fester Form, beispielsweise als Pulver oder flüssig als Schmelze erfolgen. Die Temperatur bei der Zugabe des p-Toluidins zur Schwefelsäure hat auf den weiteren Ablauf der Reaktion keinen Einfluß. Für den Fall einer Temperatursteigerung, die über den erfindungsgemäß bei der Oleumzugabe einzuhaltenden Temperaturbereich hinausgeht, muß vor Beginn der Oleumzugabe entsprechend gekühlt werden. Es ist daher zweckmäßig, die Temperatur während der Zugabe des p-Toluidins zur $H_2SO_4$ in dem Bereich zu halten, in dem anschließend die Oleumzugabe erfolgen soll.

Der Gehalt an freiem $SO_3$ im Oleum beträgt zweckmäßigerweise 20-65 Gew.-%, bevorzugt wird ein Oleum mit einem $SO_3$-Gehalt von 60-65 Gew.-% eingesetzt.

Erfindungsgemäß wird so viel Oleum zu dem in $H_2SO_4$ gelösten p-Toluidin zugegeben, daß pro Mol p-Toluidin 1-3 Mol, bevorzugt 1,1-2,5 Mol, besonders bevorzugt 1,2-2 Mol und ganz besonders bevorzugt 1,3-1,8 Mol $SO_3$ eingesetzt werden. Während der Zugabe des Oleums wird erfindungsgemäß eine Temperatur von 10-55 °C, bevorzugt 15-50 °C, besonders bevorzugt 20-45 °C eingehalten. Beginnt man die Oleumzugabe im unteren Teil des angegebenen Temperaturbereichs, so ist es möglich, während der Oleumzugabe die Temperatur bis in den oberen Teil des angegebenen Temperaturbereiches, gegebenenfalls in mehreren Schritten, anzuheben.

Nach der Oleumzugabe kann das Reaktionsgemisch, insbesondere bei noch vorhandenen größeren Mengen an p-Toluidin, noch einer Nachreaktion unterworfen werden. Das Vorhandensein von noch nicht umgesetztem p-Toluidin kann beispielsweise durch Dünnschichtchromatographie an einer Probe des Reaktionsgemisches festgestellt werden. Die Nachreaktion kann im genannten Bereich von 10-55 °C durchgeführt werden. Es ist jedoch zulässig, die Nachreaktion auch im Bereich von 55-80 °C durchzuführen. Für den Fall der Einstellung dieser Temperatur von 55-80 °C für die Nachreaktion geschieht dies nach der Zugabe von mindestens 50 % des gesamten Oleums, bevorzugt erst nach vollständiger Zugabe des Oleums, so daß gegebenenfalls mindestens 50 % des gesamten Oleums bei 10-55 °C und der Rest bei 55-80 °C zugegeben werden. Auch innerhalb der Nachreaktionszeit kann die Temperatur innerhalb der angegebenen Temperaturbereiches von 10-55 °C, gegenenfalls auch von 55-80 °C, in mehreren Schritten angehoben werden.

Zur Aufarbeitung wird das Reaktionsgemisch mit Wasser versetzt. Wasser kann hierbei als solches, als Eis/Wassergemisch oder in Form von zerkleinertem Eis eingesetzt werden. Ebenso kann das Reaktionsgemisch zum Wasser in einer der angegebenen Formen gegeben werden. Nach dem Versetzen des Reaktionsgemisches mit Wasser fällt die p-Toluidin-2-säure aus und kann durch übliche Methoden, wie Filtration oder Abzentrifugieren, gewonnen werden. Die Menge des zugesetzten Wassers in einer der angegebenen Formen wird hierbei so berechnet, daß neben der auskristallisierten p-Toluidin-2-säure als wäßrige Phase eine Dünnsäure mit einem Gehalt an Schwefelsäure von 10-60 Gew.-%, bevorzugt 20-50 Gew.-% und ganz besonders bevorzugt 30-40 Gew.-%, bezogen auf die gesamte wäßrige Phase, entsteht.

Die Nachreaktion kann im genannten Bereich von 10-55 °C durchgeführt werden. Es ist jedoch zulässig, die Nachreaktion auch im Bereich von 55-80 °C durchzuführen.

Die von der wäßrigen Phase abgetrennte p-Toluidin-2-säure hat eine Reinheit bezüglich der unerwünschten 3-Säure von mehr als 99 %, bevorzugt mehr als 99,5 % und in vielen Fällen mehr als 99,95 %. Diese Isomerenreinheit der erhaltenen 2-Säure wurde durch Hochdruck-Flüssigkeitschromatographie (HPLC) ermittelt, wo sich die 2- und die 3-Sulfonsäure durch verschiedene Retentionszeiten unterscheiden und gut abtrennen lassen. Die Konstitution der in Frage kommenden 2- bzw. 3-Sulfonsäure des p-Toluidins wurde durch Elementaranalyse, dünnschichtchromatographischen Vergleich nach Diazotierung und Kupplung mit einer Kupplungskomponente gesichert. In Kenntnis der weiter oben diskutierten Literatur war die hohe Selektivität der erfindungsgemäß hergestellten p-Toluidin-2-säure nicht vorhersehbar. Die ausschließliche Isolierung der gewünschten 2-Sulfonsäure ohne zusätzliche Reinigungsstufe, wie Waschen oder Umkristallisieren, ist daher überraschend.

p-Toluidin-2-sulfonsäure findet beispielsweise Verwendung in Antihelmintika für Haustiere (FR Addn. 84 259), in Farbstoffen als Diazokomponente (SU 191 017), als Zusatz zur Bildung kristallisationsfähiger Thermoplaste aus Polyolefinen und Polyamiden (DE-OS 2 002 489) und in optischen Aufhellern (Yuki Gosei Kagaku Kyokai Shi *1972*, 30, 449).

Beispiel 1

In 100 ml vorgelegte 100 %ige Schwefelsäure wurden innerhalb von 1 1/2 Stunden bei 20-30 °C 107 g (1 Mol) p-Toluidin portionsweise eingetragen. Anschließend ließ man in etwa 2 1/2 Stunden 20-30 °C 221 ml 20 %iges Oleum zulaufen, heizte nach beendeter Oleumzugabe den Reaktionsansatz auf 65 °C und hielt ihn 1 Stunde bei dieser Temperatur. Der Ansatz wurde auf 817 ml Wasser gegeben, wobei die Temperatur auf 80 °C anstieg und die p-Toluidin-2-säure ausfiel. Anschließend wurde auf 20 °C abgekühlt und die ausgefallene Sulfonsäure abgesaugt.

Man erhielt 210 g feuchte p-Toluidin-2-säure mit einem Gehalt von 80,0 % ; dies entspricht einer Ausbeute von 90 %, bezogen auf das eingesetzte p-Toluidin. Der Gehalt an p-Toluidin liegt unter 0,1 Gew.-% ; der Gehalt an p-Toluidin-3-säure liegt unterhalb der Nachweisgrenze durch Dünnschichtchromatographie.

Beispiel 2

In 640 g 100 %ige Schwefelsäure wurden bei Raumtemperatur 139,2 g p-Toluidin (99,5 %ig = 1,292 Mol) geschmolzen zugetropft. Die Temperatur wurde durch Wasserkühlung bei 30-40 °C gehalten. Nach beendeter Toluidinzugabe wurde 15 Minuten bei 30 °C nachgerührt. Anschließend wurden 224 g 65 %iges Oleum bei 30-40 °C in einer Stunde zugetropft. Man rührte 1 Stunde bei 40 °C und 1 Stunde bei 60 °C nach. Das Sulfiergemisch wurde auf 1 160 ml Wasser ohne Kühlung ausgetragen und anschließend auf 20 °C abgekühlt. Die Suspension wurde über eine Glasfritte abgesaugt. Die kristalline, hellgelbe p-Toluidin-2-säure hatte ein Feuchtgewicht von 302,3 g und einen Gehalt von 75,5 Gew.-% an p-Toluidin-2-säure.

Die Ausbeute, bezogen auf eingesetztes p-Toluidin, betrug 94,4 %. Der Gehalt an p-Toluidin-3-säure lag unter 0,05 Gew.-%.

**Patentansprüche**

1. Verfahren zur Herstellung von p-Toluidin-2-sulfonsäure durch Reaktion von $SO_3$-haltiger Schwefelsäure (Oleum) mit in Schwefelsäure gelöstem p-Toluidin, dadurch gekennzeichnet, daß man zur Auflösung pro Mol p-Toluidin 1,5-10 Mol Schwefelsäure einsetzt und so viel Oleum bei 10-55 °C zugibt, daß pro Mol p-Toluidin 1-3 Mol

freies SO$_3$ eingesetzt werden.

2. Verfahren nach Anspruch 1, dadurch gekennzeichnet, daß man pro Mol p-Toluidin 1,7-8 Mol Schwefelsäure einsetzt.

3. Verfahren nach Anspruch 1 und 2, dadurch gekennzeichnet, daß 1,1-2,5 Mol freies SO$_3$ pro Mol p-Toluidin eingesetzt werden.

4. Verfahren nach Anspruch 1 und 2, dadurch gekennzeichnet, daß 1,2-2 Mol freies SO$_3$ pro Mol p-Toluidin eingesetzt werden.

5. Verfahren nach Ansprüchen 1 bis 4, dadurch gekennzeichnet, daß das Oleum bei 15-50 °C zugegeben wird.

6. Verfahren nach Ansprüchen 1 bis 4, dadurch gekennzeichnet, daß das Oleum bei 20-45 °C zugegeben wird.

7. Verfahren nach Ansprüchen 1 bis 6, dadurch gekennzeichnet, daß man das Reaktionsgemisch einer Nachreaktion bei 10-80 °C unterwirft.

8. Verfahren nach Ansprüchen 1 bis 7, dadurch gekennzeichnet, daß der Temperaturbereich von 55-80 °C für die Nachreaktion nach Zugabe von mindestens 50 % des gesamten Oleums eingestellt wird.

9. Verfahren nach Ansprüchen 1 bis 7, dadurch gekennzeichnet, daß der Temperaturbereich von 55-80 °C für die Nachreaktion nach Zugabe des gesamten Oleums eingestellt wird.

10. Verfahren nach Ansprüchen 1 bis 9, dadurch gekennzeichnet, daß man die Temperatursteigerung in mehreren Schritten vornimmt.

## Claims

1. Process for the preparation of p-toluidine-2-sulphonic acid by reacting SO$_3$-containing sulphuric acid (oleum) with p-toluidine dissolved in sulphuric acid, characterised in that for the dissolution 1.5-10 moles of sulphuric acid are used per mole of p-toluidine and such an amount of oleum is added at 10-55 °C that 1-3 moles of free SO$_3$ are used per mole of p-toluidine.

2. Process according to Claim 1, characterised in that 1.7-8 moles of sulphuric acid are used per mole of p-toluidine.

3. Process according to Claim 1 and 2, characterised in that 1.1-2.5 moles of free SO$_3$ are used per mole of p-toluidine.

4. Process according to Claim 1 and 2, characterised in that 1.2-2 moles of free SO$_3$ are used per mole of p-toluidine.

5. Process according to Claims 1 to 4, characterised in that the oleum is added at 15-50 °C.

6. Process according to Claims 1 to 4, characterised in that the oleum is added at 20-45 °C.

7. Process according to Claims 1 to 6, characterised in that the reaction mixture is subjected to an after-reaction at 10-80 °C.

8. Process according to Claims 1 to 7, characterised in that the temperature range of 55-80 °C for the after-reaction is adjusted after at least 50 % of the total oleum has been added.

9. Process according to Claims 1 to 7, characterised in that the temperature range of 55-80 °C for the after-reaction is adjusted after all the oleum has been added.

10. Process according to Claims 1 to 9, characterised in that the temperature is increased in more than one step.

## Revendications

1. Procédé de préparation de l'acide p-toluidine-2-sulfonique par réaction de l'acide sulfurique contenant SO$_3$ (oléum) avec la p-toluidine dissoute dans l'acide sulfurique, caractérisé en ce que, pour dissoudre la p-toluidine, on utilise de 1,5 à 10 moles d'acide sulfurique par mole de p-toluidine, et on ajoute à une température de 10 à 55 °C de l'oléum en quantité correspondant à 1 à 3 moles de SO$_3$ libre par mole de p-toluidine.

2. Procédé selon la revendication 1, caractérisé en ce que l'on utilise de 1,7 à 8 moles d'acide sulfurique par mole de p-toluidine.

3. Procédé selon les revendications 1 et 2, caractérisé en ce que l'on utilise de 1,1 à 2,5 moles de SO$_3$ libre par mole de p-toluidine.

4. Procédé selon les revendications 1 et 2, caractérisé en ce que l'on utilise de 1,2 à 2 moles de SO$_3$ libre par mole de p-toluidine.

5. Procédé selon les revendications 1 à 4, caractérisé en ce que l'on ajoute l'oléum à une température de 15 à 50 °C.

6. Procédé selon les revendications 1 à 4, caractérisé en ce que l'on ajoute l'oléum à une température de 20 à 45 °C.

7. Procédé selon les revendications 1 à 6, caractérisé en ce que l'on soumet le mélange de réaction à une réaction complémentaire à une température de 10 à 80 °C.

8. Procédé selon les revendications 1 à 7, caractérisé en ce que l'on règle dans l'intervalle de température de 55 à 80 °C pour la réaction complémentaire après addition d'au moins 50 % de l'oléum total.

9. Procédé selon les revendications 1 à 7, caractérisé en ce que l'on règle dans l'intervalle de température de 55 à 80 °C pour la réaction complémentaire après addition de la totalité de l'oléum.

10. Procédé selon les revendications 1 à 9, caractérisé en ce que l'on augmente la température en plusieurs paliers.